# EUROPÄISCHE PATENTSCHRIFT

(11) **EP 3 586 914 B1**
(45) Veröffentlichungstag und Bekanntmachung des Hinweises auf die Patenterteilung: **06.03.2024**
(21) Anmeldenummer: 18202077.6
(22) Anmeldetag: 23.10.2018
(51) Int. Cl.: A61N 5/06, A61N 1/372, A61N 1/05, A61N 1/362, A61B 5/00, A61N 5/10, A61N 1/368, A61N 5/073

(54) **VORRICHTUNG ZUR AKTIVIERUNG VON ZELLSTRUKTUREN MITTELS ELEKTROMAGNETISCHER ENERGIE**
DEVICE FOR ACTIVATING OF CELL STRUCTURES BY MEANS OF ELECTROMAGNETIC ENERGY
DISPOSITIF D'ACTIVATION DES STRUCTURES CELLULAIRES AU MOYEN DE L'ÉNERGIE ÉLECTROMAGNÉTIQUE

(30) Priorität: 25.06.2018 DE 102018115180
(43) Veröffentlichungstag der Anmeldung: 01.01.2020
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Biotronik Corporate Services SE

(56) Entgegenhaltungen:
- US-A1- 2012 290 025
- US-A1- 2013 231 721
- US-A1- 2013 274 838
- US-A1- 2015 224 315
- US-B2- 9 622 809

## Beschreibung

Die Erfindung beschreibt eine Vorrichtung zur Aktivierung von Zellstrukturen mittels elektromagnetischer Energie.

Seit langem bekannt sind Stimulatoren zur Anregung von menschlichem oder tierischem Gewebe durch elektrischen Strom. Elektrische Stimulation von Nervengewebe bewirkt die Aktivierung von Aktionspotentialen, die in der medizinischen Therapie und Diagnostik genutzt wird. Beispiele für implantierbare Stimulatoren sind u.a. implantierbare Herzschrittmacher und -Defibrillatoren, Rückenmarkstimulatoren, Vagusnervstimulatoren, Hirnschrittmacher.

Elektrische Stimulationsvorrichtungen sind galvanisch an menschliches oder tierisches Gewebe gekoppelt und messen elektrische Potentiale und/oder stimulieren das Gewebe durch elektrische Stromabgabe zur Auslösung von Aktionspotentialen. Nachteile der elektrischen Stimulation ergeben sich unter anderem durch die notwendige galvanische Verbindung zum Gewebe. Die direkte Kontaktierung Stimulationselektroden mit dem Gewebe und Einkopplung von Strom können z.B. Nekrosen, elektrische Nachpotentiale, die die Sensingfunktionen limitieren, Crosstalk (d.h. Interferenzen bei paralleler Stimulation über verschiedene Stimulationspfade), ungewollte externe Induktion von Therapieströmen (z.B. bewirkt durch die hochfrequenten Magnetfelder innerhalb eines Magnetresonanztomografen), eventuelle Schmerzereignisse, Elektroporation oder ungewollte Stimulation in der Nähe befindlichen Gewebes (z.B. kann bei Herzstimulatoren der Nervus Phrenicus ungewollt mitstimuliert werden) verursachen. Weitere Nachteile ergeben sich durch die mit dem Therapiestrom verbundenen Einrichtungen zur Stromgenerierung, die aufwändig und voluminös sein können (z.B. Ladeschaltung bei einem implantierbaren Kardioverter-Defibrillator) und Limitationen in der Erreichbarkeit des Zielgewebes aufweisen.

Seit einigen Jahren ist die genetische Manipulation von Gewebe zur Generierung einer Erregbarkeit mittels elektromagnetischer Wellen Gegenstand der wissenschaftlichen Diskussion und Forschung. Dabei ist es gelungen, Zielgewebe genetisch so zu verändern, dass mittels elektromagnetischen Wellen Aktionspotentiale evoziert werden können.

Eine dieser Methoden wird als optogenetische Manipulation bezeichnet. Diese wird beispielsweise beschrieben in
- Lung MS, Pilowsky P, Goldys EM., "Activation of the Mammalian cells by using light-sensitive ion channels." Methods Mol Biol. 2012;875:241-51; die Publikation beschreibt optogenetische Gewebemanipulation zum Zwecke der Auslösung von Aktionspotentialen mittels Licht im sichtbaren Spektrum;
- Wang et al. "Optogenetic Control of Heart Rhythm by Selective Stimulation of Cardiomyocytes Derived from Pnmt+ Cells in Murine Heart", Januar 2017 in Scientific Reports.

US 2012/0290025 A1 beschreibt Techniken zur Abgabe von genetischem Material an eine Zielgewebestelle eines Patienten über ein elektrodenloses implantierbares medizinisches Gerät, das eine Pumpe und ein Element zur Abgabe von Stimulationsenergie umfasst.

US 2009/0088680 A1 beschreibt ein katheterbasiertes System zur Einbringung eines Lichtleiters in das Herz, wobei das Herzgewebe zuvor mittels optogenetischer Manipulation behandelt wurde. Um den Lichtleiter in das Herzinnere zu befördern, wird ein Gefäßzugang beim Patienten gelegt, ein Katheter wird in das Herz vorgeschoben und der Lichtleiter durch den Katheter in das Herzinnere gebracht. Über den Lichtleiter wird Licht in das Herz geleitet und eine endokardiale Stimulation des manipulierten Gewebes durchgeführt.

Ein Nachteil der in US 2009/0088680 A1 beschriebenen Methode besteht darin, dass sie durch einen Arzt ambulant durchgeführt werden und der Lichtleiter muss von einem externen Gerät betrieben werden muss. Nachteile entstehen auch durch den venösen Gefäßzugang und die Katheterisierung wie zum Beispiel Infektionen und Gefäßverschlüsse.

US 2013/231721 A1 beschreibt ein implantierbares Gerät zur Nervenstimulation mittels Licht.

Die Aufgabe der Erfindung ist es, in einer dauerhaft implantierbaren Vorrichtung Aktionspotentiale an vorbehandelten tierischen und/oder menschlichen erregbaren Zellstrukturen zu evozieren, modulieren, terminieren und/oder zu inhibieren ohne dass es einer galvanischen Einprägung elektrischem Stromes bedarf.

Eine weitere Aufgabe der vorliegenden Erfindung ist die Bereitstellung einer Vorrichtung und eines Systems, um an vorbehandelten tierischen und/oder menschlichen erregbaren Zellstrukturen zu evozieren, modulieren, terminieren und/oder zu inhibieren, ohne dass es der Implantation einer Elektroden- oder Sondenleitung bedarf.

Eine weitere Aufgabe der Erfindung ist es, besagte Vorrichtung bereitzustellen, die eine Therapieerfolgskontrolle ermöglicht.

Eine Aufgabe der vorliegenden Erfindung ist die Bereitstellung einer Vorrichtung und eines Systems, das die genannten Nachteile nicht aufweist.

Eine weitere Aufgabe der vorliegenden Erfindung besteht in der Bereitstellung einer Vorrichtung und eines Systems für die Stimulation von Gewebe mittels elektromagnetischen Wellen zur Auslösung von Aktionspotentialen, wobei die Vorrichtung eine dauerhafte Stimulation ermöglicht, ohne dass ein permanenter Gefäßzugang, externe Geräte oder eine ambulante Sitzung beim Arzt erforderlich ist.

Die Aufgabe wird erfindungsgemäß durch die Merkmale des unabhängigen Anspruchs 1 gelöst. Günstige Ausgestaltungen und Vorteile der Erfindung ergeben sich aus den weiteren Ansprüchen und der Beschreibung.
- Figur 1: Schematische Darstellung eines Ausführungsbeispiels der erfindungsgemäßen Stimulationsvorrichtung im implantierten Zustand mit einem externen Gerät
- Figur 2: Schematische Darstellung einer Methode zur Behandlung einer Bradykardie mittels optischer Stimulation
- Figur 3: Schematische Darstellung einer Methode zur Behandlung einer tachykarden Herzrhythmusstörung in Form einer ventrikulären oder atrialen Tachykardie
- Figur 4: Schematische Darstellung einer Methode zur Behandlung eines Vorhofflimmerns
- Figur 5: Schematische Darstellung einer Methode zur Behandlung eines Kammerflimmerns
- Figur 6: Schematische Darstellung einer Methode zur Anwendung der kardialen Resynchronisationstherapie mittels elektromagnetischer Wellen
- Figur 7: Schematische Darstellung einer Neurostimulationsmethode mittels elektromagnetischer Wellen
- Figur 8: Schematische Darstellung einer Ausführungsform eines erfindungsgemäßen implantierbaren Stimulators
- Figur 9: Implantierbarer Stimulator gemäß Figur 8 mit einer alternativen Fixiervorrichtung
- Figur 10: Blockschaltbild des implantierbaren Stimulators gemäß einem Ausführungsbeispiel der Erfindung

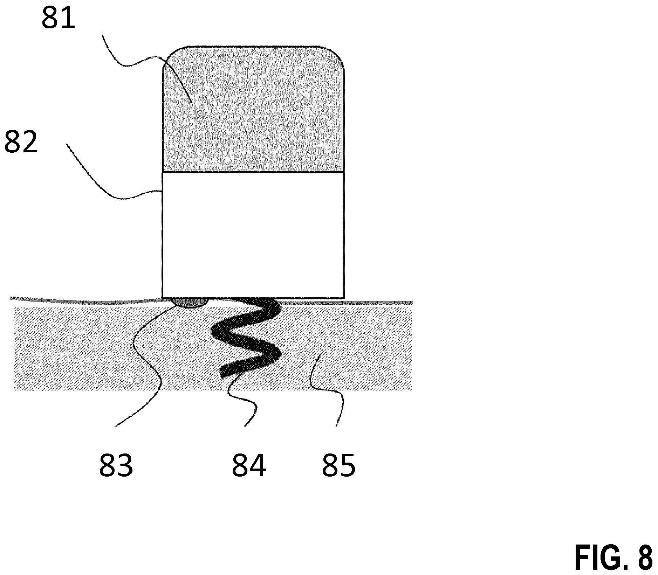

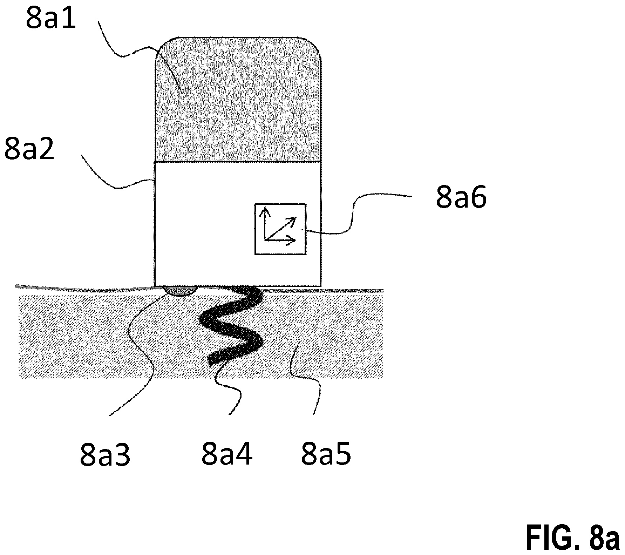

- Figur 8a: Schematische Darstellung einer Ausführungsform der erfindungsgemäßen Stimulationsvorrichtung mit Therapieerfolgskontrolle

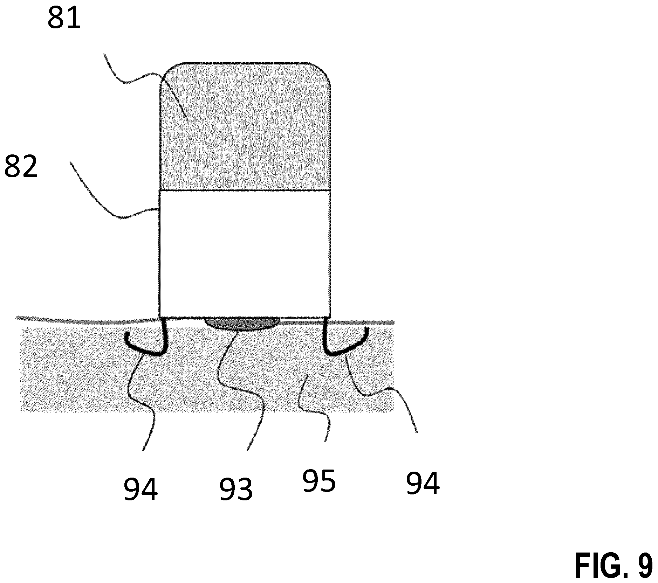

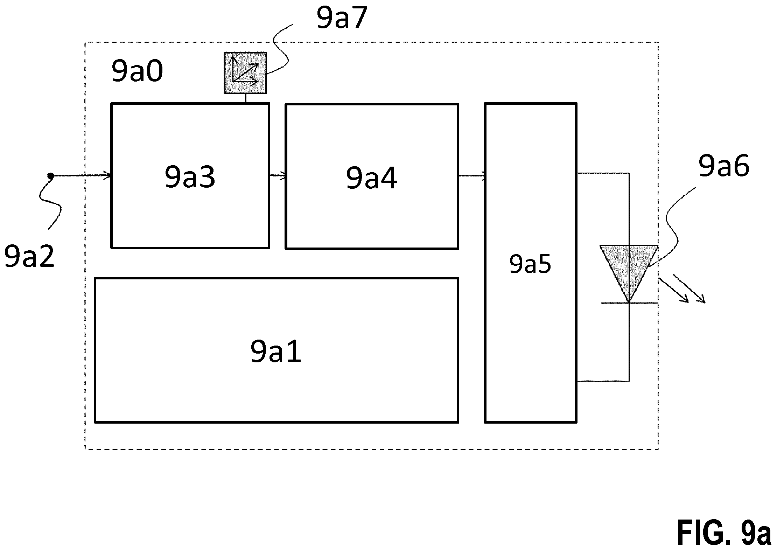

- Figur 9a: Blockschaltbild der erfindungsgemäßen Stimulationsvorrichtung gemäß eines Ausführungsbeispiels

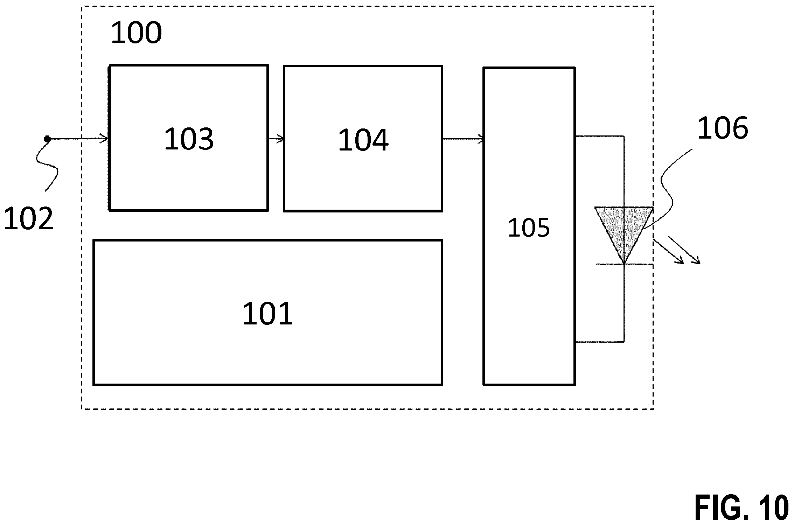

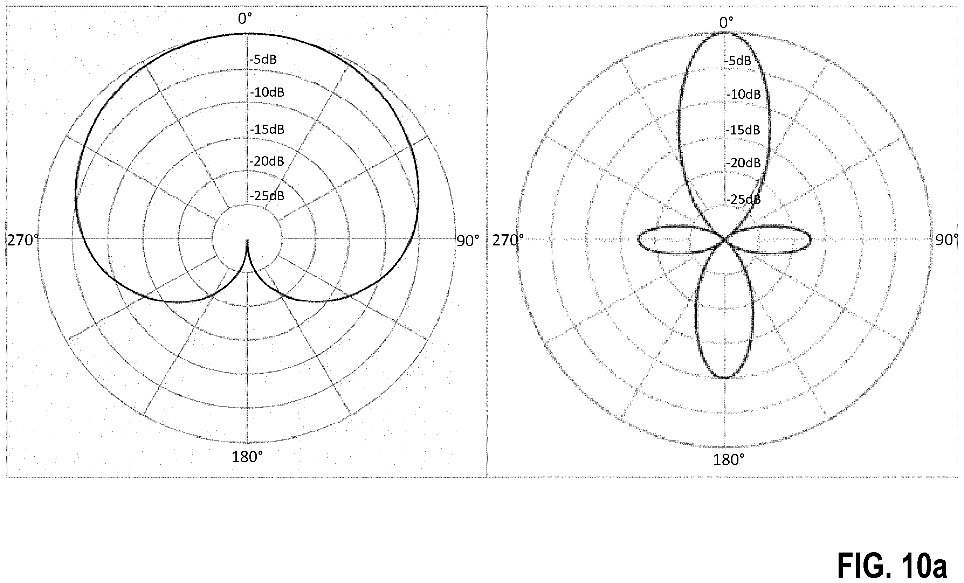

- Figur 10a: zeigt beispielhaft zweidimensionale Charakteristiken elektromagnetischer Strahlers mit räumlich selektiver Wirkung.

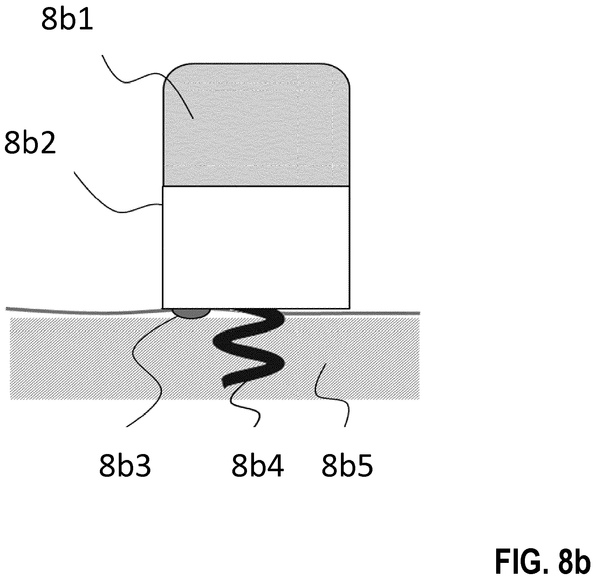

- Figur 8b: Schematische Darstellung einer Ausführungsform mit Defibrillationsfunktion

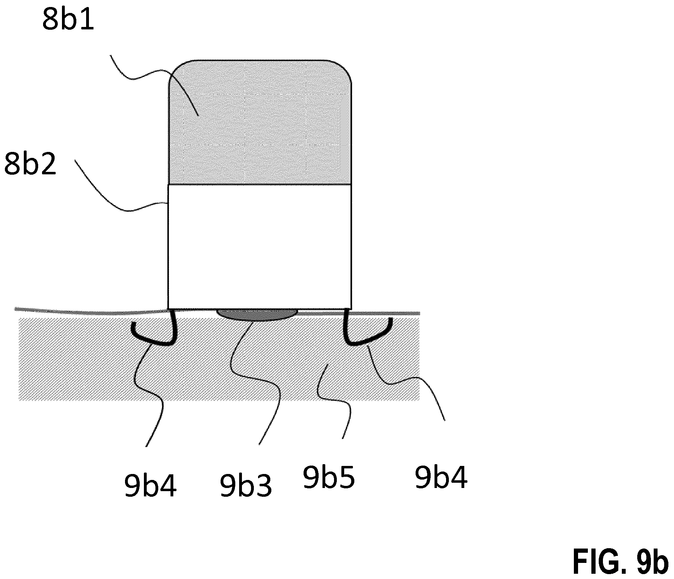

- Figur 9b: Implantierbarer Stimulator gemäß Figur 8b mit einer alternativen Fixiervorrichtung
- Figur 10a: Blockschaltbild der Stimulationsvorrichtung gemäß eines Ausführungsbeispiels nach Figur 8b

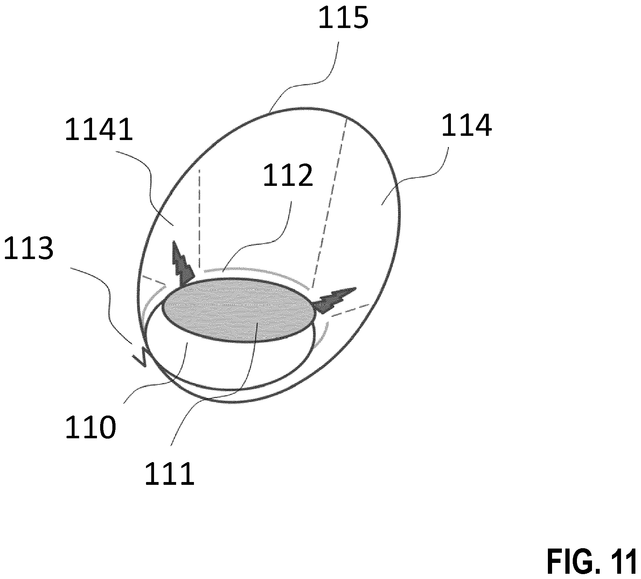

- Figuren 11 bis 14: zeigen Realisierungen des Stimulationssystems mit Selektionsmittel, das dazu ausgelegt ist, den Bereich des besagten Gewebes für die Stimulation zu wählen gemäß Ausführungsformen

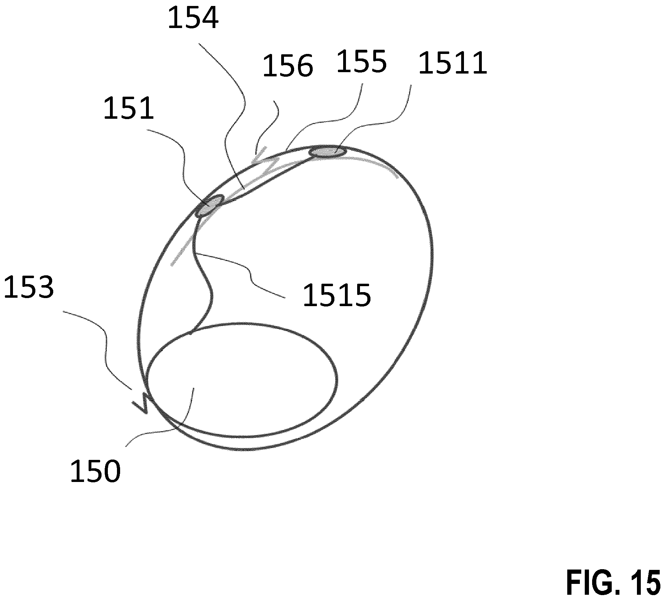

- Figur 15: Realisierung des Stimulationssystems mit Selektionsmittel, das dazu ausgelegt ist, den Bereich des besagten Gewebes für die Stimulation zu wählen, wobei das Stimulationssystem über Träger verfügt

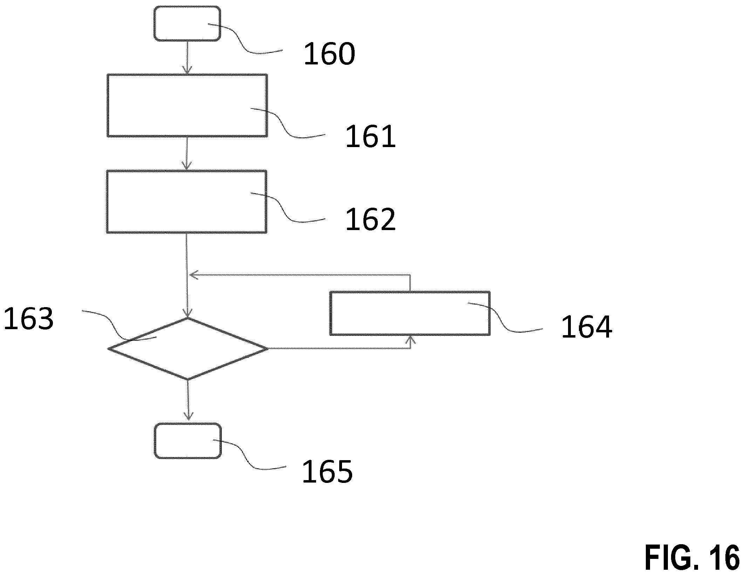

- Figur 16: Ablaufplan der Methode für das Stimulationssystem gemäß Ausführungsbeispielen

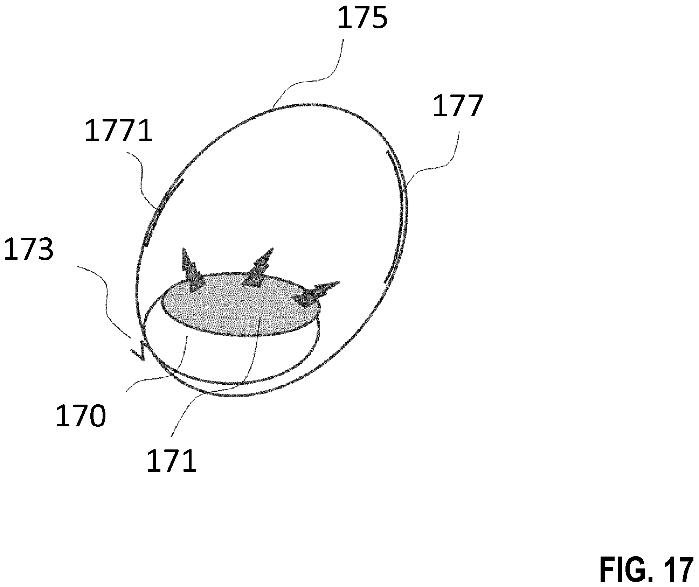

- Figur 17: Schematische Darstellung der lokalen Vorbehandlung zur Erzeugung empfindsamer Bereiche für elektromagnetische Strahlung

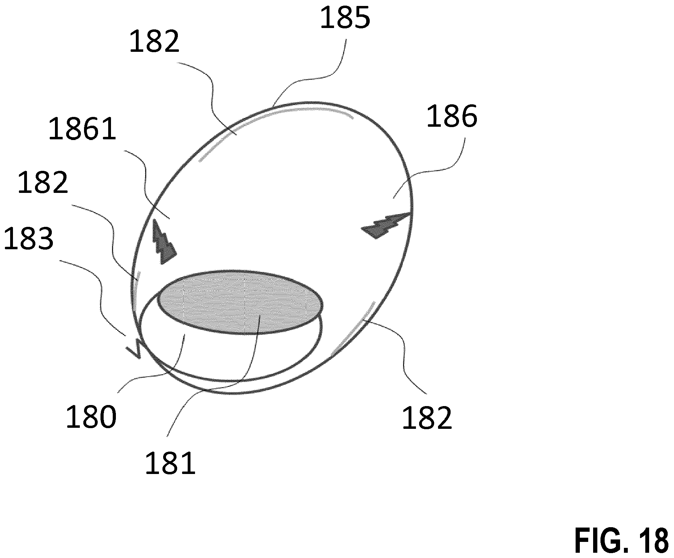

- Figur 18: Schematische Darstellung der lokalen Vorbehandlung zur Erzeugung einer Maskierung ggf. empfindsamer Bereiche für elektromagnetische Strahlung.

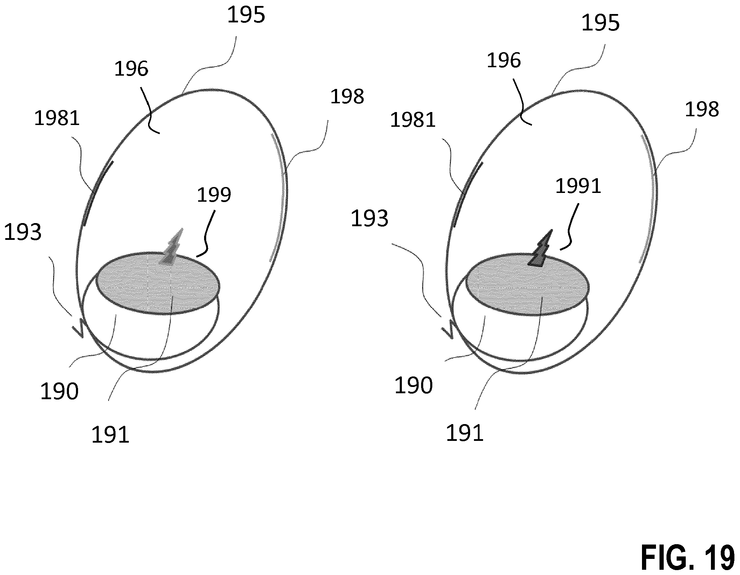

- Figur 19: Beispiel mit lokal vorbehandelten Bereichen für die Stimulation mittels elektromagnetischer Strahlung

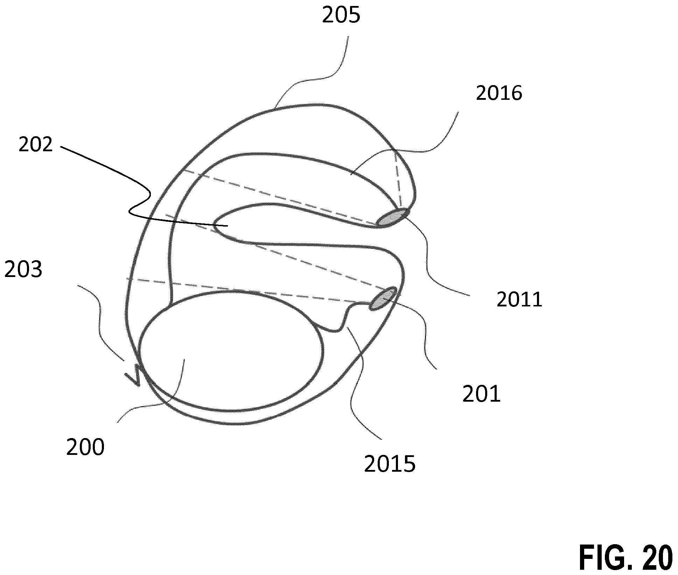

- Figur 20: Schematische Darstellung der selektiven Therapie durch Platzierung der Aktuatoren, so dass anatomische Beschaffenheiten des Gewebes zur Abschattung genutzt werden.

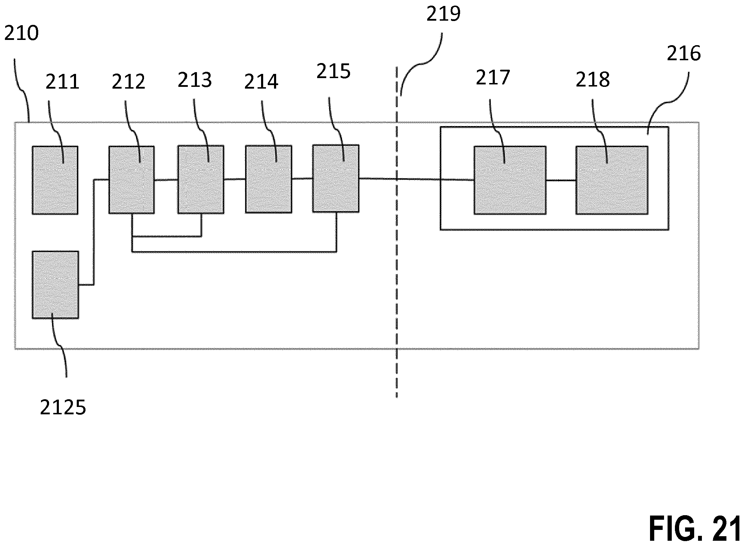

- Figur 21: Blockschaltbild eines erfindungsgemäßen Stimulators gemäß einer Ausführungsform

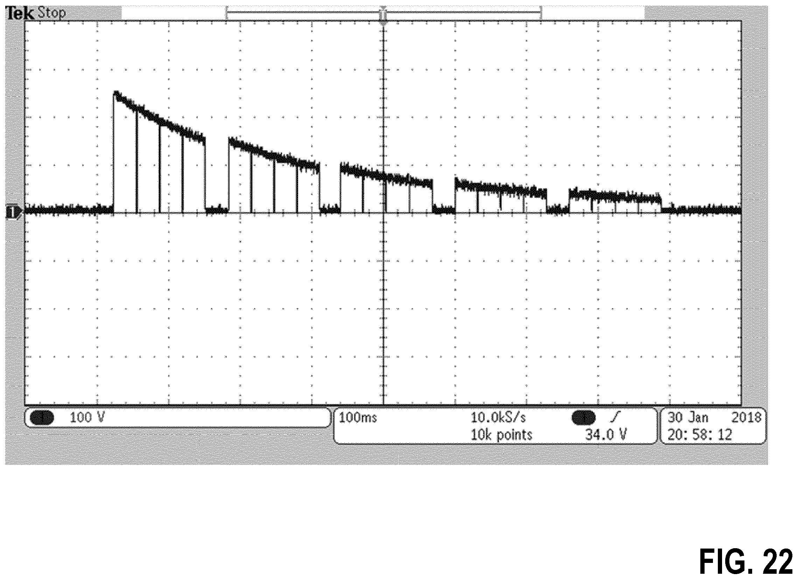

- Figur 22: Schematische Darstellung der Pulsentladung eines ICDs, die einen Stimulator mit Therapiemöglichkeit mittels elektromagnetischer Strahlung zur Abgabe einer gepulsten Therapie ansteuert.

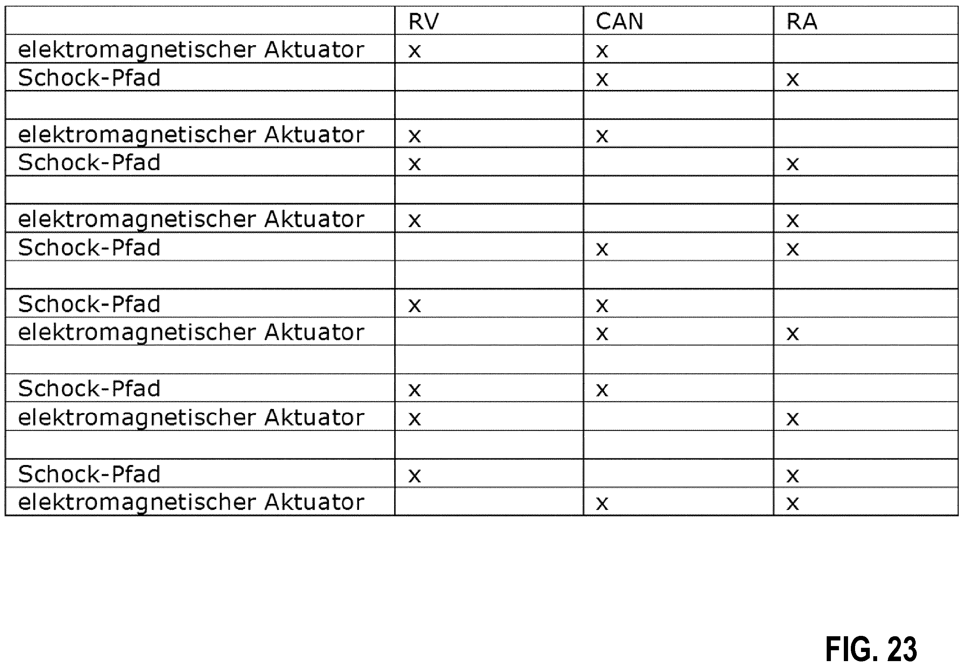

- Figur 23: Tabelle, aufzeigend beispielhafte Kombinationen von Stimulations- und Schockvektoren zwischen rechtem Ventrikel, Gehäuse der Stimulationsvorrichtung und rechtem Atrium.

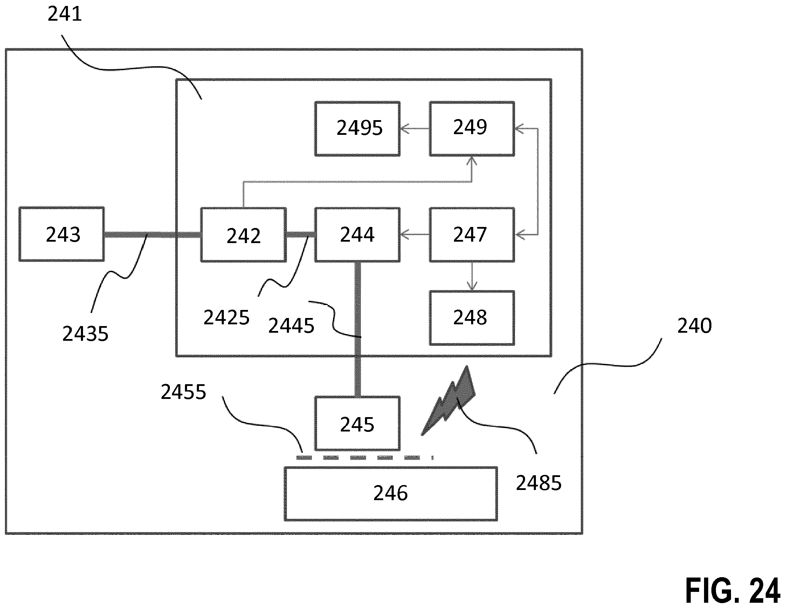

- Figur 24: Schematische Darstellung der Vorrichtung gemäß einer Ausführungsform, bei der die Vorrichtung ein Applikationsmittel zur Abgabe einer Substanz an Gewebe umfasst.

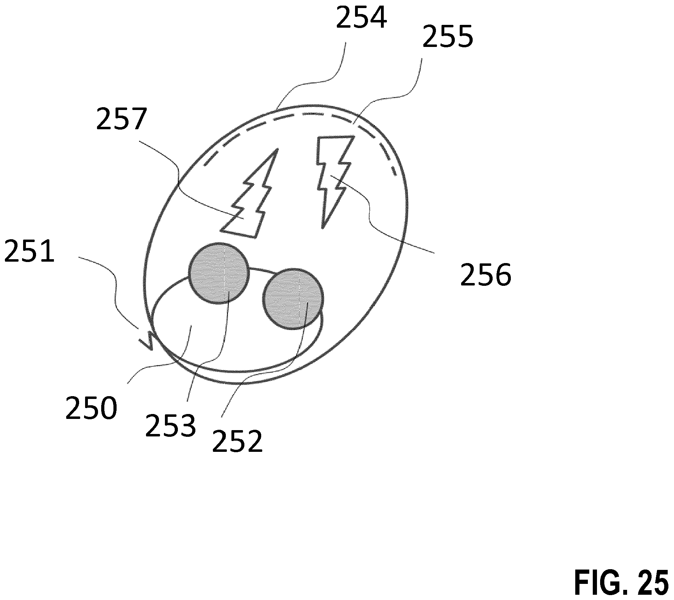

- Figur 25: Schematische Darstellung der implantierbaren Vorrichtung nach einem Ausführungsbeispiel, wobei die Vorrichtung mit einer Fixiereinrichtung am Gewebe fixiert ist

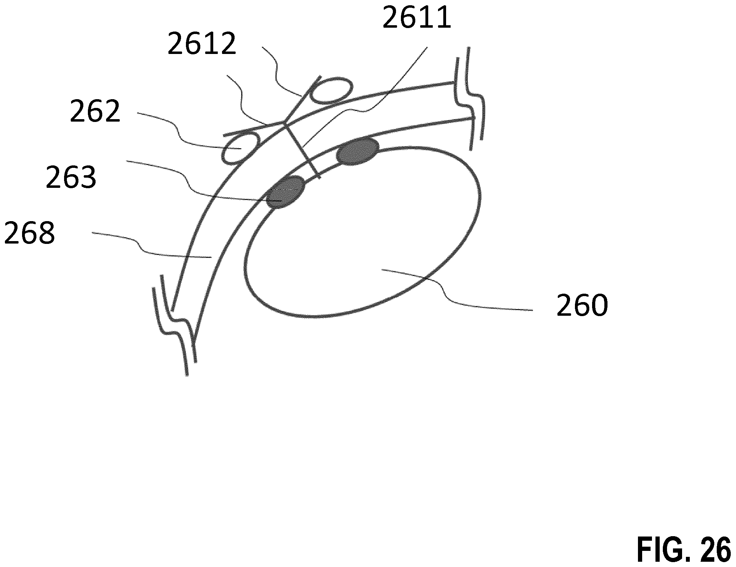

- Fig. 26: Schematische Darstellung der Vorrichtung gemäß einem Realisierungsbeispiel. Die Vorrichtung verfügt über Aktuatoren bzw Sensoren für elektromagnetische Strahlung, die in der Fixiereinheit eingebaut sind.

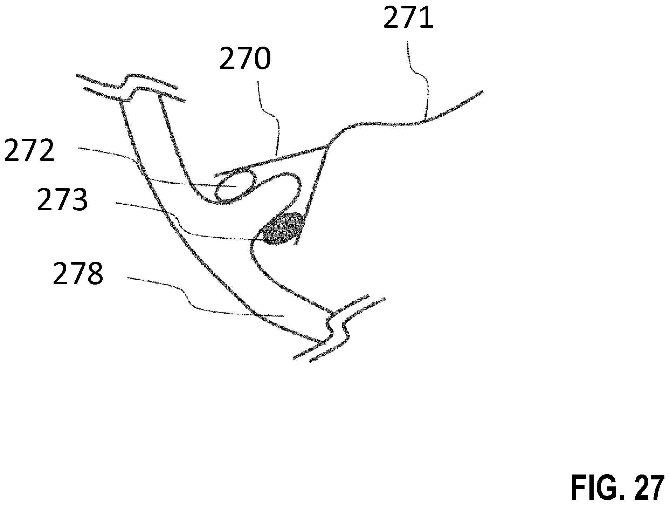

- Fig. 27: Schematische Darstellung der Vorrichtung gemäß einem weiteren Realisierungsbeispiel. Die Vorrichtung verfügt über Aktuatoren bzw Sensoren für elektromagnetische Strahlung, die in der Fixiereinheit eingebaut sind.

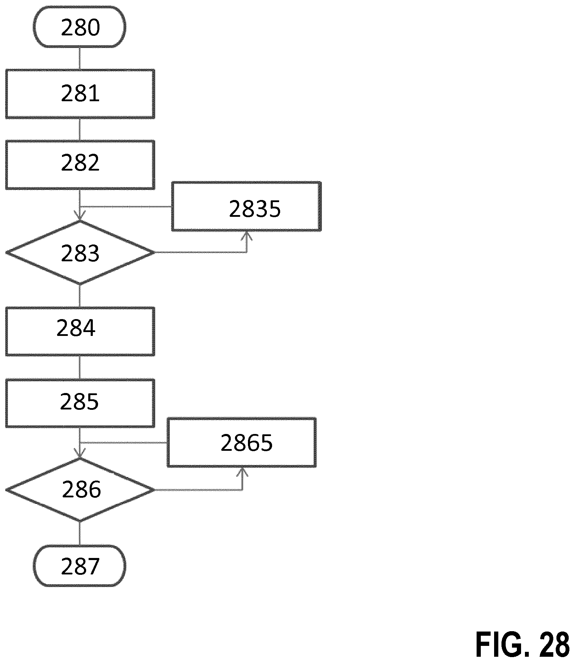

- Figur 28: zeigt beispielhaft einen Implantationsablauf.

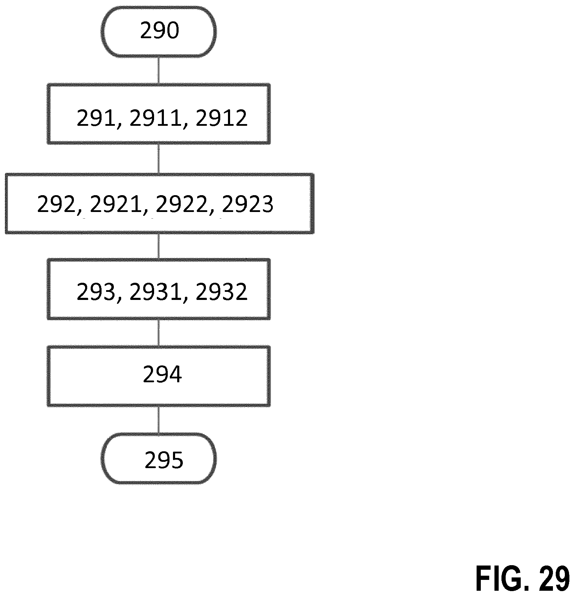

- Figur 29: zeigt beispielhaft einen Signalverarbeitungsablauf

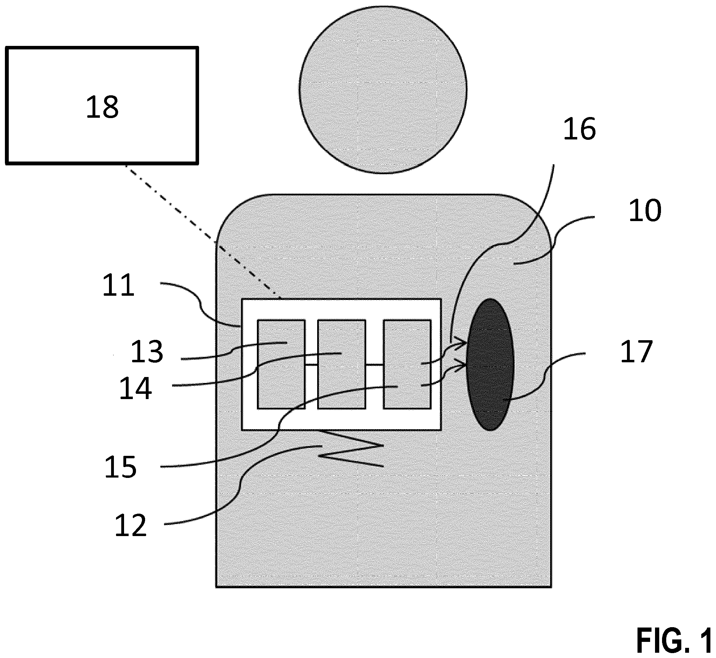

In Figur 1 ist ein erfindungsgemäßer implantierbarer Stimulator dargestellt. Dieser wird in das Körpergewebe des Patienten 10 implantiert und umfasst aus einem hermetisch versiegelten Gehäuse 11. Diese ist hier mit einer Fixiervorrichtung 12 im Körpergewebe verankert. Das Implantatgehäuse 11 beinhaltet eine Energiequelle 13, eine Steuereinheit 14 und einen Aktuator 15, der ausgebildet ist, eine elektromagnetische Strahlung 16 z.B. im Bereich des sichtbaren Lichtes an ein zuvor beispielsweise mittels genetischer, speziell optogenetischer Manipulation vorbehandeltes Körpergewebe abgeben zu können und beispielsweise ein Aktionspotential zu evozieren, d.h. eine Stimulation mittels elektromagnetsicher Strahlung auszulösen Zusätzlich kann das Implantat mittels eines externen Geräts 18, z.B. einer Telemetrieeinheit ausgelesen und/oder programmiert werden.

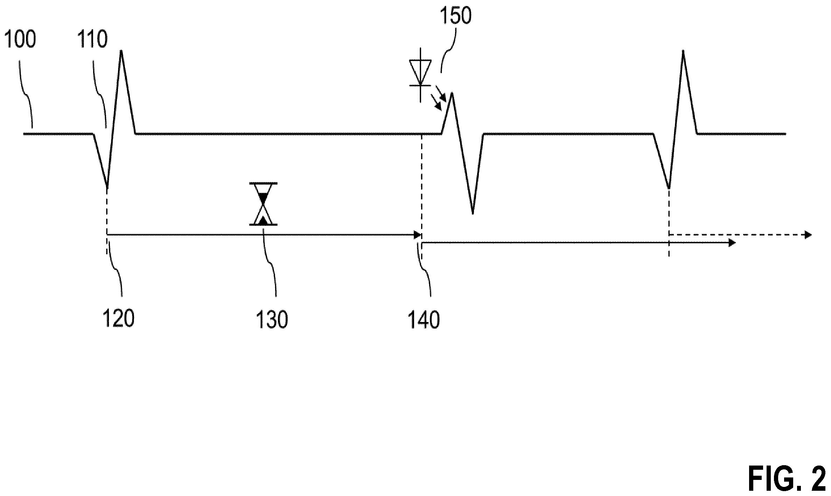

In der Figur 2 ist eine Methode zur Behandlung einer Bradykardie mittels Stimulation von genetisch manipuliertem Gewebe, bevorzugt optogenetisch manipuliertem Gewebe, illustriert. Im schematisierten EKG 100 ist zunächst eine reguläre Herzaktion 110 in Form eines intrinsischen QRS-Komplex dargestellt. Dieser wir bei dem vorgeschlagenen Verfahren zunächst registriert 120 und anschließend ein Zeitintervall 130 gestartet, dass einer erwarteten Herzrate entspricht. Läuft dieses Erwartungsintervall ab 140, ohne dass erneut ein intrinsischer QRS-Komplex registriert wird, so wird eine optische Stimulation 150 ausgelöst, die im vorzugsweise optogenetisch vorbehandelten Myokardgewebe einen stimulierten QRS-Komplex auslöst.

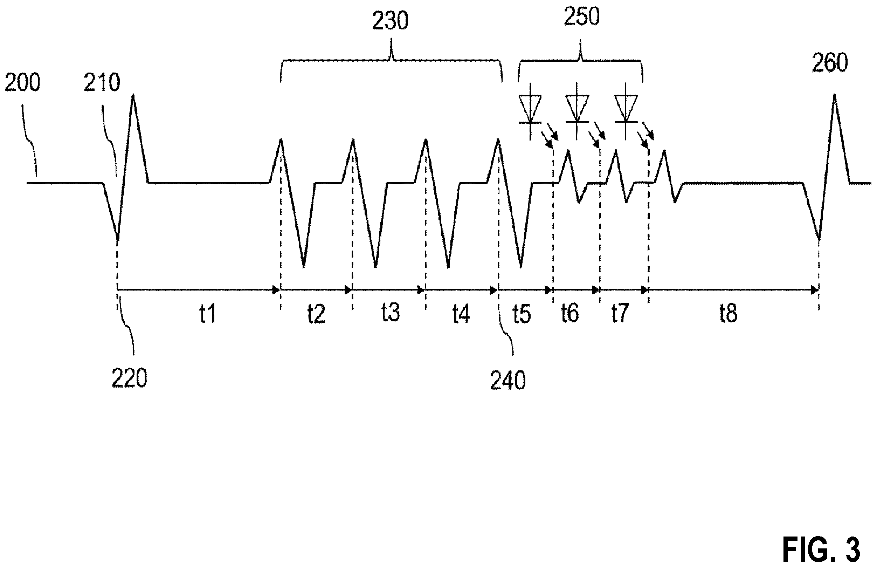

In der Figur 3 ist eine Methode zur Behandlung einer tachykarden Herzrhythmusstörung in Form einer ventrikulären oder atrialen Tachykardie illustriert. Im schematischen EKG 200 ist zunächst eine reguläre Kontraktion 210 einer Herzkammer dargestellt. Zur Überwachung des Herzrhythmus wir jede dieser Kontraktionen registriert und startet eine Zeitmessung bis zur nächsten registrierten Kontraktion (11... t8). Im dargestellten Beispiel setzt nun eine zu schnelle Herzrate in Form einer Tachykardie 230 ein, die zunächst über einige Zyklen registriert und bestätigt wird 240. Nach Bestätigung 240 wird eine vorbestimmte optische Stimulationssequenz 250 an das vorzugsweise vorbehandelte Myokard abgegeben, welche eine Reihe von stimulierten Erregungen verursacht, die in der Regel die Tachykardie beenden und einen regulären Rhythmus wieder herstellen 260.

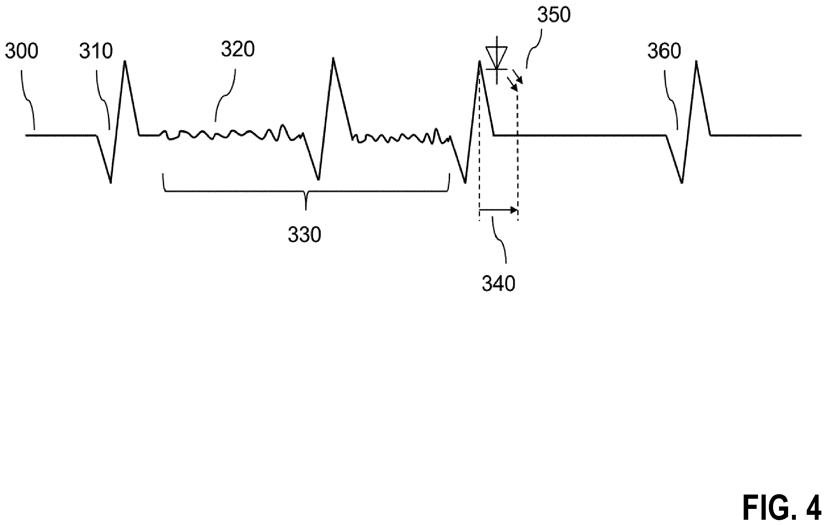

In der Figur 4 ist eine Methode zur Behandlung eines Vorhofflimmerns illustriert. Im schematischen EKG 300 ist zunächst eine QRS-Komplex in Sinusrhythmus 310 dargestellt. Anschließend setzt ein Vorhofflimmern 320 spontan ein. Dieses wird zunächst registriert und bestätigt 330. Mit der Bestätigung erfolgt die Abgabe einer optischen Stimulation 350 der Vorhöfe des vorbehandelten Herzens, jedoch synchronisiert und zeitversetzt zu einem QRS-Komplex (Kardioversion). Hiermit wird das Vorhofflimmern terminiert und ein regulärer Sinusrhythmus wird wieder hergestellt 360.

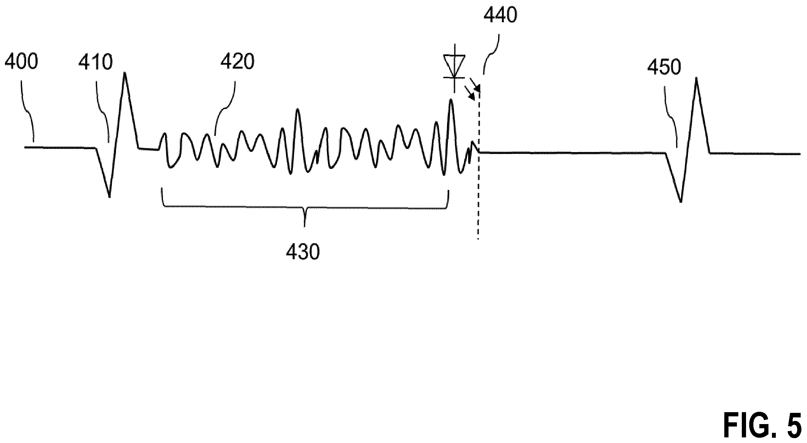

In der Figur 5 ist eine Methode zur Behandlung eines Kammerflimmerns illustriert. Im schematischen EKG 400 ist zunächst eine QRS-Komplex in Sinusrhythmus 410 dargestellt. Anschließend setzt spontan Kammerflimmern ein 420, welches zunächst registriert und anschließend bestätigt wird 430. Mit der Bestätigung wird ein großflächige optische Stimulation 440 im Bereich der vorzugsweise optogenetisch vorbehandelten Ventrikel ausgelöst, welche das Kammerflimmern terminiert und einen Sinusrhythmus 450 wiederherstellt.

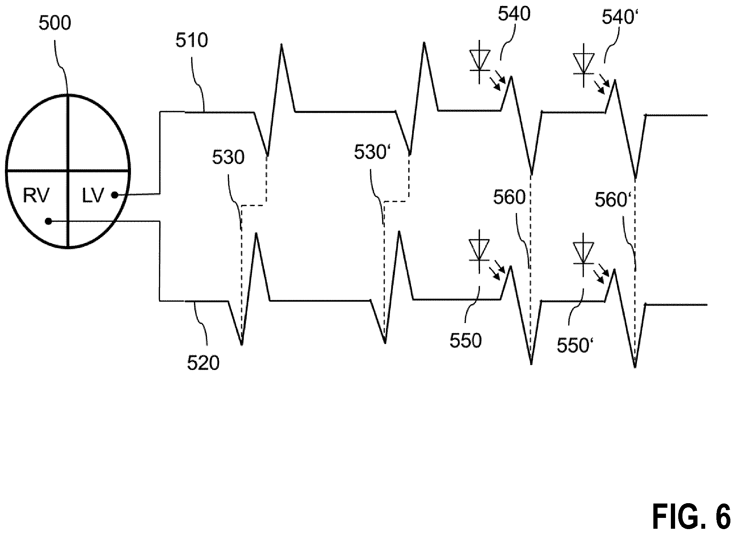

In der Figur 6 ist eine kardiale Resynchronisationsmethode illustriert zur Stimulation von genetisch manipuliertem Gewebe, bevorzugt optogenetisch manipuliertem Gewebe.

Dargestellt sind schematisch die intrakardialen EKG-Ableitungen aus beiden Ventrikeln des Herzen 500: Linker Ventrikel (LV; 510) und rechter Ventrikel (RV, 520). Die EKG Ableitungen zeigen zunächst eine zeitliche Verzögerung 530 der linksventrikulären Herzaktionen gegenüber der rechtsventrikulären Herzaktionen. Dieser Versatz ist Ausdruck eines sogenannten Linksschenkelblocks, der bei fortgeschrittener struktureller Schädigung des Herzmuskels einer Therapie in Form einer Resynchronisation beider Ventrikel bedarf. Diese Resynchronisation erfolgt hier mittels simultaner oder quasisimultaner optischer Stimulation 540,550 beider Ventrikel des Herzens, so dass zeitgleich eine mechanische Kontraktion 560 beider Ventrikel dauerhaft hergestellt wird.

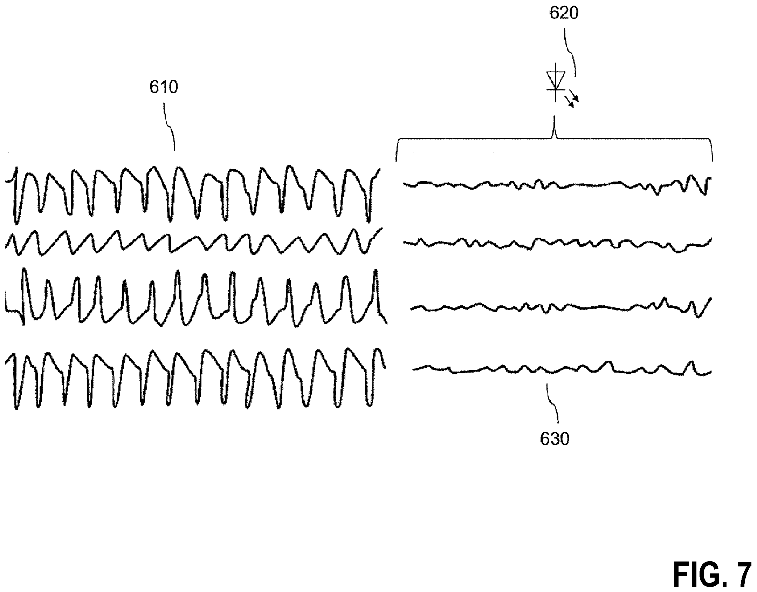

In der Figur 7 ist beispielhaft eine Neurostimulation von genetisch manipuliertem Gewebe, bevorzugt optogenetisch manipuliertem Gewebe, illustriert. Im gezeigten Beispiel ist auf der linken Seite ein EEG-Ausschnitt 610 eines epileptischen Anfalls abgebildet. Zu dessen Behandlung wird eine optische Stimulationssequenz 620 an eine oder mehrere vorzugsweise optogenetisch vorbehandelte Hirnregionen abgegeben, um eine Terminierung des epileptischen Anfalls herbeizuführen 630. Diese Darstellung soll die Methode der optischen Neurostimulation stellvertretend für alle weiteren Applikationen illustrieren, so z.B. Rückenmarksstimulation, weitere Applikationen der Deep Brain Stimulation oder kortikale Stimulation, isolierte Nervenstimulation, Muskelstimulation etc.

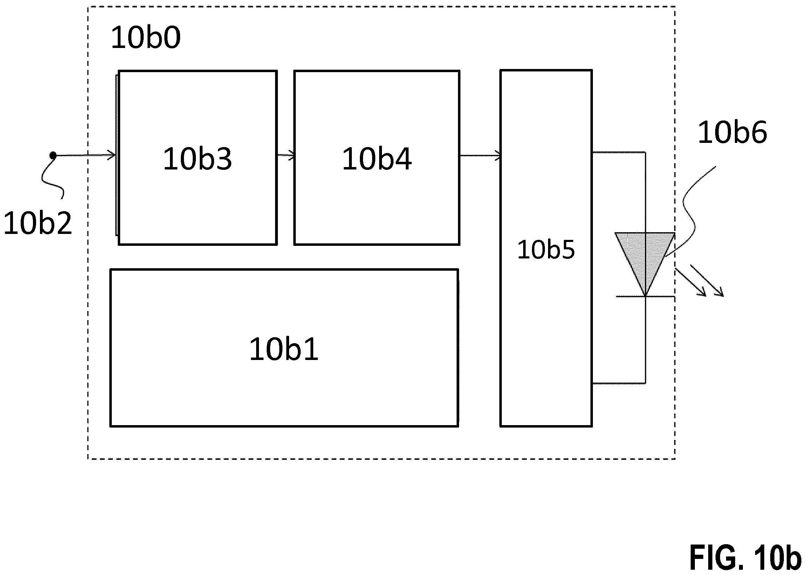

In der Figur 8 ist eine mögliche Ausführungsform eines erfindungsgemäßen implantierbaren Stimulators dargestellt. Dieser besteht aus einer Batterie 81 und einer Implantatselektronik 82, die die Komponenten des Blockschaltbildes aus Figur 10 beinhaltet, beides in einem hermetischen Gehäuse untergebracht. An der Unterseite des Gehäuses ist eine Lichtquelle 83 angebracht, die ebenfalls hermetisch versiegelt ist, jedoch so, dass das Frequenzspektrum dieser Lichtquelle die hermetische Versiegelung durchdringen und das Zielgewebe -hier Myokard- 85 erregen kann. Die Fixierung des Implantates erfolgt hier mit einer Helix 84 im Myokard 85.

In der Figur 9 ist der implantierbare Stimulator aus Figur 8 bestehend aus einer Batterie 81 und einer Implantatselektronik 82 dargestellt, jedoch mit einer alternativen Fixiervorrichtung 94, 94` in Form von seitlich angebrachten Widerhaken aus z.B. Nitinol.

An der Unterseite des Gehäuses ist eine Lichtquelle 93 angebracht, die hermetisch versiegelt ist, derart, dass das Frequenzspektrum dieser Lichtquelle die hermetische Versiegelung durchdringen und das Zielgewebe -hier Myokard- 95 erregen kann.

In der Figur 10 ist das Blockschaltbild des implantierbaren Stimulators 100 dargestellt. Dieses Umfasst eine Energiequelle 101, ein Sensorinterface 102 zur Wahrnehmung eines eine Herzaktion repräsentierenden Merkmals, verbunden mit einer Wahrnehmungseinheit 103, die wiederum die Wahrnehmung von Herzaktionen an die angeschlossene Steuereinheit 104 signalisiert. Diese Steuereinheit 104 ist mit einem Therapiegenerator 105 verbunden, welcher bei Erhalt eines Triggersignals von der Steuereinheit 104 eine an den Therapiegenerator angeschlossene LED 106 anregt und damit ein das Myokardgewebe stimulierendes Lichtsignal aussendet. Der Therapiegenerator 105 kann dabei das Lichtsignal in seiner Intensität, Dauer, Signalform und Farbe variieren.

In der Figur 8a ist eine mögliche Ausführungsform eines erfindungsgemäßen implantierbaren Stimulators mit Therapieerfolgskontrolle dargestellt. Dieser besteht aus einer Batterie 8a1 und einer Implantats-Elektronik 8a2, die die Komponenten des Blockschaltbildes aus Figur 9a beinhaltet, beides in einem hermetischen Gehäuse untergebracht. An der Unterseite des Gehäuses ist eine Lichtquelle 8a3 angebracht, die ebenfalls hermetisch versiegelt ist, jedoch so, dass das Frequenzspektrum dieser Lichtquelle die hermetische Versiegelung durchdringen und das Zielgewebe -hier Myokard- 8a5 erregen kann. Die Fixierung des Implantates erfolgt hier mit einer Helix 8a4 im Myokard 8a5. In diesem Ausführungsbeispiel umfasst die Implantats-Elektronik 8a2 zusätzlich ein 3D-Beschleunigungssensor 8a6, der zur Therapieerfolgskontrolle derart eingesetzt wird, dass nach jeder optischen Stimulation ausgewertet wird, ob eine Beschleunigung des am Myokard fixierten Implantates festgestellt wird. In diesem Falle gilt die Stimulation als effektiv, da eine Kontraktion des Herzgewebes zur Beschleunigung führt. Bleibt die Beschleunigung aus, gilt die Stimulation als ineffektiv und wird z.B. mit höherer Amplitude wiederholt oder alternativ eine ineffektive Stimulation an ein Fernüberwachungssystem signalisiert.

In der Figur 9a ist das Blockschaltbild des implantierbaren Stimulators 9a0 dargestellt. Dieses Umfasst eine Energiequelle 9a1, ein Sensorinterface 9a2 zur Wahrnehmung eines eine Herzaktion repräsentierenden Merkmals, verbunden mit einer Wahrnehmungseinheit 9a3, die wiederum die Wahrnehmung von Herzaktionen an die angeschlossene Steuereinheit 9a4 signalisiert. Diese Steuereinheit 9a4 ist mit einem Therapiegenerator 9a5 verbunden, welcher bei Erhalt eines Triggersignals von der Steuereinheit 9a4 eine an den Therapiegenerator angeschlossene LED 9a6 anregt und damit ein das Myokardgewebe stimulierendes Lichtsignal aussendet. Der Therapiegenerator 9a5 kann dabei das Lichtsignal in seiner Intensität, Dauer, Signalform und Farbe variieren.

Die Wahrnehmungseinheit 9a3 ist ferner mit ein 3D-Beschleunigungssensor 9a7 verbunden, der zur Therapieerfolgskontrolle derart eingesetzt wird, dass nach jeder optischen Stimulation ausgewertet wird, ob eine Beschleunigung des am Myokard fixierten Implantates festgestellt wird. In diesem Falle gilt die Stimulation als effektiv, da eine Kontraktion des Herzgewebes zur Beschleunigung führt. Bleibt die Beschleunigung aus, gilt die Stimulation als ineffektiv und wird z.B. mit höherer Intensität, Dauer, alternativer Signalform oder anderer Farbe wiederholt.

Figur 10a zeigt beispielhaft zweidimensionale Charakteristiken elektromagnetischer Strahlers mit räumlich selektiver Wirkung.

In der Figur 8b ist eine mögliche Ausführungsform eines implantierbaren Defibrillators dargestellt. Dieser besteht aus einer Hochleistungs-LED 8b 1 und einer verkapselten Implantatselektronik nebst Energiequelle 8b2, die die Komponenten des Blockschaltbildes aus Figur 3 beinhaltet, beides in einem hermetischen Gehäuse untergebracht. An der Unterseite des Gehäuses ist eine weitere lokale Lichtquelle 8b3 angebracht, die derart angebracht und dimensioniert ist, dass diese nur einen lokale Depolarisation im vorbehandelten Myokard 8b5 auszulösen vermag. Die Fixierung des Implantates erfolgt hier mit einer Helix 8b4 im Myokard 8b5. Die Hochleistungs-LED 8b1 ist derart dimensioniert, das diese zum Zwecke der Defibrillation nahezu das ganze Herz "durchscheinen" kann, so dass eine gleichzeitige Depolarisation aller erregbaren Myokardzellen im Augenblick der Defibrillation möglich ist.

In der Figur 9b ist der implantierbare Stimulator aus Figur 8b dargestellt, jedoch mit einer alternativen Fixiervorrichtung 9b4, 9b4` in Form von seitlich angebrachten Widerhaken aus Nitinol. An der Unterseite des Gehäuses ist eine Lichtquelle 9b3 angebracht, die hermetisch versiegelt ist, derart, dass das Frequenzspektrum dieser Lichtquelle die hermetische Versiegelung durchdringen und das Zielgewebe -hier Myokard- 9b5 erregen kann.

In der Figur 10b ist das Blockschaltbild des implantierbaren Stimulators 10b0 gemäß Figur 8b dargestellt. Dieses Umfasst eine Energiequelle 10b1, ein Sensorinterface 10b2 zur Wahrnehmung eines eine Herzaktion repräsentierenden Merkmals, verbunden mit einer Wahrnehmungseinheit 10b3, die wiederum die Wahrnehmung von Herzaktionen an die angeschlossene Steuereinheit 10b4 signalisiert. Diese Steuereinheit 10b4 ist mit einem Therapiegenerator 10b5 verbunden, welcher bei Erhalt eines Triggersignals von der Steuereinheit 10b4 eine an den Therapiegenerator angeschlossene LED 10b6 anregt und damit ein das Myokardgewebe stimulierendes Lichtsignal aussendet. Der Therapiegenerator 10b5 kann dabei das Lichtsignal in seiner Intensität, Dauer, Signalform und Farbe variieren.

In der Figur 11 wird eine typische Realisierung dargestellt. Das Implantat 110 ist mit einer Fixiereinrichtung 113 an Organgewebe befestigt und verfügt über einen elektromagnetischen Strahler 111 der mittels der Maske 112 maskiert ist, um nur innerhalb der Wirkkegel 114 und 1141 elektromagnetische Energie auf das zu therapierende Gewebe 115 einstrahlen zu lassen.

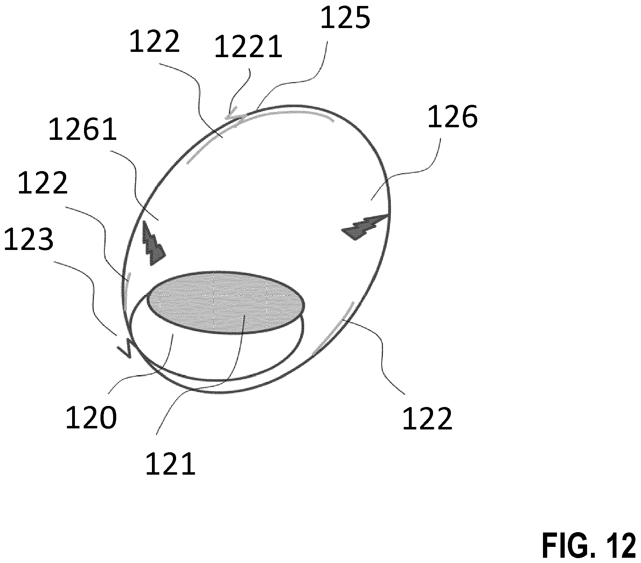

In der Figur 12 ist die Realisierung offenbart, bei der die Maske 122 eine zweite Einheit darstellt und unabhängig vom Implantat mit einer Fixiervorrichtung 1221 befestigt ist, um zu verhindern dass elektromagnetische Energie in die abgedeckten Geweberegionen eindringt. Das Implantat 120 ist mit einer Fixiereinrichtung 123 an Organgewebe befestigt und verfügt über einen elektromagnetischen Strahler 121 der mittels der Maske 122 maskiert ist. Die Therapie wirkt nur in den nichtmaskierten Regionen 126 und 1261 auf das zu therapierende Gewebe 125.

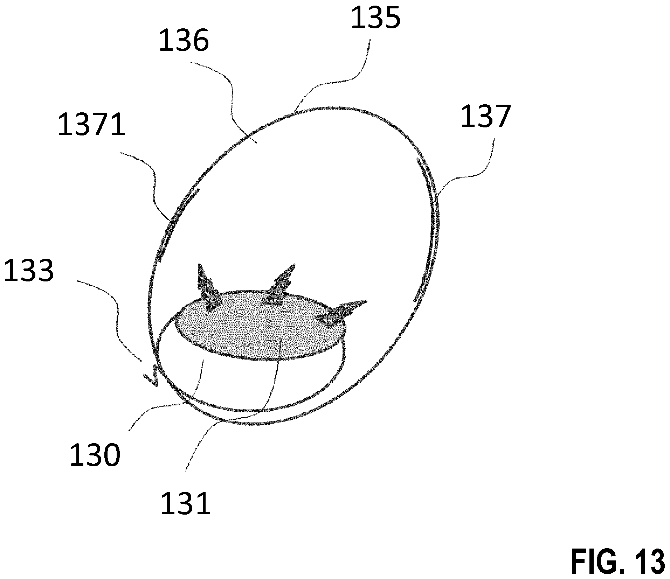

Figur 13 zeigt eine Realisierung mit lokal vorbehandeltem Gewebe. Der Wirkbereich 136 der Strahlung erreicht zwar das gesamte Gewebe 135, wirkt jedoch nur den in den Regionen 137 und 1371.- Das Implantat 130 ist mit einer Fixiereinrichtung 133 an Organgewebe befestigt und verfügt über einen elektromagnetischen Strahler 131. Die Therapie wirkt nur in den vorbehandelten Regionen 137 und 1371 auf das zu therapierende Gewebe 135.

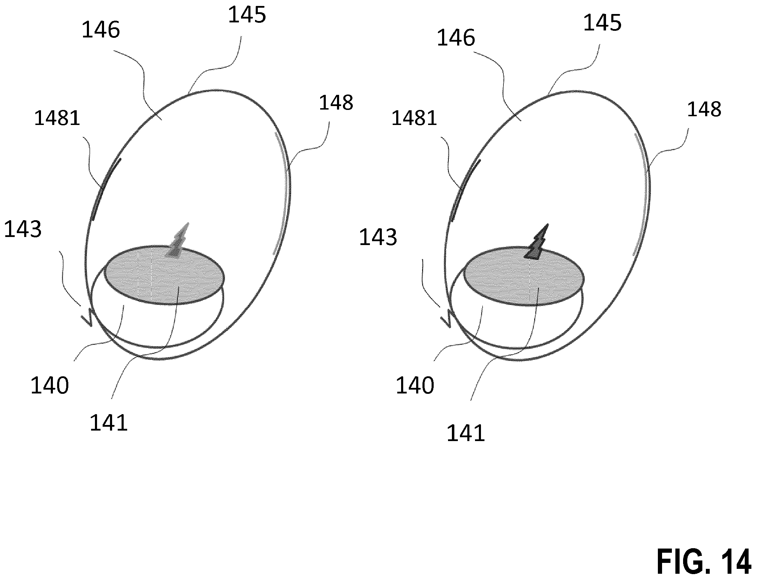

Figur 14 offenbart eine Lösung mit lokal frequenzspezifisch (oder polarisationsspezifisch) reagierendem oder dahingehend vorbehandeltem Gewebe. Je nach Frequenz(band) bzw. Polarisation des Strahlers zeigt nur die eine 148 oder die andere 1481 Region Wirkung. Der Wirkbereich 146 der Strahlung erreicht zwar das gesamte Gewebe 145, wirkt jedoch nur den in den Regionen 148 und 1481. Das Implantat 140 ist mit einer Fixiereinrichtung 143 an Organgewebe befestigt und verfügt über einen elektromagnetischen Strahler 141.

Figur 15 zeigt eine Lösung mit distal zum Implantatgehäuse liegenden Strahlern 151 und 1511 die entweder direkt (nicht gezeigt) oder mittels eines Trägers 154 mit Fixierung 156 an des zu therapierende Gewebe angebracht sind. Dabei übernimmt der Träger auch die Rolle einer Maske und schattet das restliche Organ ab. Die Strahler werden vom Implantat 150 über eine Leitung 1515 versorgt. Alternativ kann das Implantat selbst die Rolle eines Trägers übernehmen. Die Strahler sind dann direkt am Gehäuse befestigt. Das Implantat 150 ist mit einer Fixiereinrichtung 153 an Organgewebe befestigt.

In der Figur 16 ist der Ablaufplan der Methode für das Stimulationssystem gezeigt. Nach Start 160 erfolgt die Vorbehandlung 161 gefolgt von der Implantation und Befestigung des Stimulators 162. Danach wird der Test 163 der Therapiewirksamkeit durchgeführt. Ist dieser nicht erfolgreich wird im Vorgang 164 nachgebessert. Anderenfalls ist die Methode beendet 165.

Figur 17 zeigt das Gewebe175 mit den Vorbehandelten Bereichen 177 und 1771. Der Stimulator 170 mit elektromagnetischem Aktuator 171 wird mit der Fixierung 173 am Gewebe befestigt.

Figur 18 zeigt das Gewebe185 mit den durch Vorbehandlung maskierten Bereichen182. Es sind somit nur die Bereiche 186 und 1861 für die vom Aktuator 181 abgegebenen Strahlung stimulierbar. Das Implantat 180 ist mit einer Fixiereinrichtung 183 an Organgewebe befestigt und verfügt über einen elektromagnetischen Strahler 181.

Figur 19 zeigt ein Beispiel mit lokal vorbehandelten Bereichen 198 und 1981 die jedoch für unterschiedlichen Frequenzen der elektromagnetischen Strahlung empfindsam sind. Die selektive Wirkung der Therapie kommt dadurch zustande, dass der Aktuator 191 elektromagnetische Strahlung einmal auf der einen Frequenz 199 und ein anderes Mal auf einer anderen Frequenz 1991 abgibt. Der Wirkbereich 196 der Strahlung erreicht zwar das gesamte Gewebe 195, wirkt jedoch nur den in den Regionen 198 und 1981. Das Implantat 190 ist mit einer Fixiereinrichtung 193 an Organgewebe befestigt und verfügt über einen elektromagnetischen Strahler 191.

Figur 20 zeigt ein Beispiel für eine selektive Therapie durch Platzierung der Aktuatoren so dass anatomische Beschaffenheiten des Gewebes zur Abschattung genutzt werden. Figur 20 zeigt wie selektive Therapie erreicht wird indem durch Platzierung der Aktuatoren 201 und 2011 (mit dem Stimulator 200 verbunden durch Leitungen 2015 und 2016) anatomische Formen 202 des Gewebes 205 zur Abschattung genutzt werden. Das Implantat 200 ist mit einer Fixiereinrichtung 203 an Organgewebe befestigt.

In der Figur 21 ist das Blockschaltbild des in den Ansprüchen beschriebenen Stimulators mit optischem Therapieausgang dargestellt. Der Stimulator 210 umfasst eine Energiequelle 211, mindestens einen Energiespeicher 214, eine Vorrichtung zur Ladung 213 des Energiespeichers 214, eine Vorrichtung zur Freigabe der Energie 215. Des Weiteren ist dargestellt ein Aktuator 216, wobei der Aktuator über eine Lichtquelle 218, sowie einen Strombegrenzer 217 (z.B. Widerstand oder Diode) verfügt. Der Stimulator 210 verfügt über eine Steuereinheit 212. Stimulator 210 umfasst eine Wahrnehmungseinheit 2125. Aktuator 216 kann als eine von der übrigen erfindungsgemäßen Vorrichtung trennbare Einheit realisiert sein und über eine Schnittstelle 219 zur Kontaktierung verfügen.

In der Figur 22 ist die Pulsentladung eines ICD dargestellt, die einen optischen Therapieaktuator zur Abgabe einer gepulsten Therapie ansteuert.

In einer Ausführungsform können bereits bekannte elektrische Stimulationsvorrichtungen (z.B. Herzschrittmacher, Neuro-Stimulator) mit einem Aktuator für die Aussendung von elektromagnetischer Strahlung ergänzt werden, um eine Stimulation mittels elektromagnetischer Wellen durchzuführen. Abgesehen vom Aktuator bedarf es dabei für das Stimulationsgerät nur geringfügiger bis keinerlei Modifikationen. Figur 23 zeigt tabellarisch beispielhafte Kombinationen von Stimulations- und Schockvektoren zwischen rechtem Ventrikel RV, Gehäuse der Stimulationsvorrichtung CAN und rechtem Atrium RA. Betrachtet man exemplarisch die zweite und dritte Zeile der Tabelle, so stellt diese eine Kombination zweier Vektoren dar: ein erster Vektor von Gehäuse CAN zum rechten Ventrikel RV für die Stimulation durch einen Aktuator gemäß der Erfindung, sowie einen zweiten Schockvektor zwischen Gehäuse CAN und rechtem Atrium RA. Bei einem handelsüblichen ICD können diese elektrischen Pole realisiert sein durch eine rechtsventrikuläre Schockwendel, das ICD-Gehäuse und eine supraventrikuläre Schockwendel. Ein Vektor führt jeweils von einem ersten durch das Kreuz gekennzeichneten Pol zu einem zweiten. Die Stimulation kann durch elektromagnetische Strahlung über einen Aktuator gemäß der Erfindung durchgeführt werden, oder durch bekannte elektrische Stimulation, oder durch eine Kombination der beiden.

Figur 24 zeigt schematisch eine Vorrichtung umfassend ein Applikationsmittel zur Abgabe einer Substanz an Gewebe. Das Gerät 241 ist im Köper 240 implantiert. Es verfügt über ein Reservoir 242 für die therapeutische Substanz und Zuleitungen 2435, 2425 und 2445. Das Reservoir wird über einen Port 243 befüllt. Die Pumpe pumpt gesteuert von der Steuereinheit 247 die Substanz zur Gerät-Gewebe-Schnittstelle oder Applikationsmittel 245, die über eine optionale Maske 2455 das Organ 246 mit dieser Substanz behandelt. Eine Kontrolleinheit 249 nimmt u.a. den Reservoirstand wahr und meldet diesen über die Telemetrieeinheit 2495 nach außen. Zum Test der Wirksamkeit gibt von 247 gesteuert die Therapieeinheit 248 ein Testsignal 2485 ab. Die Reaktion wird über eine Wahrnehmungseinheit (hier nicht gezeigt) festgestellt und der Kontrolleinheit gemeldet.

Figur 25 zeigt die implantierbare Vorrichtung 250 die mit Fixiereinrichtung 251 am Gewebe 254 fixiert ist und über einen Sensor 252 zur Wahrnehmung von elektromagnetischer Strahlung 256 verfügt, die als primäre Strahlung von den zu beobachtenden (optional vorbehandelten) erregbaren Zellstrukturen 255 ausgeht, wenn diese Aktionspotentiale ausbilden.

In einer alternativen Realisierung/Anwendungsszenario verfügt die implantierbare Vorrichtung 250 über einen zusätzlichen Aktuator 253 für die Erzeugung von elektromagnetischer Strahlung 257. Diese wird von den zu beobachtenden (optional vorbehandelten) erregbaren Zellstrukturen 255 in Abhängigkeit ihrer Aktionspotentiale moduliert und kehrt als sekundäre Strahlung 256 zum Sensor 252 zurück.

In der Figur 26 wird eine Realisierung mit in der Fixiereinheit eingebauten Aktuatoren und Sensoren für elektromagnetische Strahlung offenbart. Die Fixiereinheit 261 besteht hier aus einem Schaft 2611 mit klappbaren, arretierbaren Armen 2612 die z.B. die Sensoren 262 tragen. Die Aktuatoren 263 sind gegenüberliegend z.B. am Gehäuse 260 fixiert. Bei Implantation werden die Arme in Richtung des Schaftes ausgerichtet, durch die Organwand 268 gestochen und dann umgeklappt und in der Stellung arretiert, dass sie den Aktuatoren jenseits der Wand passend gegenüberliegen

In der Figur 27 wird eine weitere Realisierung mit in der Fixiereinheit eingebauten Aktuatoren 273 und Sensoren 272 für elektromagnetische Strahlung offenbart. Dabei ist die Fixiereinheit 271 der Aktoren 273 und Sensoren 272 als Zwacke 270 ausgeführt. Eine Zuleitung 271 führt zum Gerät (nicht gezeigt).

Figur 28 zeigt beispielhaft einen Implantationsablauf.

Die dargestellten Schritte sind:
- 280 Start der Implantation
- 281 Vorbehandlung des Zielgewebes
- 282 Feststellung des geeigneten Implantationsortes
- 283 Positionierung der Vorrichtung, solange bis der geeignete Implantationsort erreicht ist
- 2835 Nachstellen der Position
- 284 Fixierung
- 285 Parametereinstellung und Start der Wahrnehmungsmethode (inkl. Aufbereitung und Analyse)
- 286 Testung
- 2865 Nachstellen der Parameter
- 287 Ende der Implantation.

Figur 29 zeigt beispielhaft einen Signalverarbeitungsablauf. Die dargestellten Schritte sind:
- 290 Start der Wahrnehmung (Messung)
- 291 Verstärkung,
- 2911 Demodulation,
- 2912 analoge Filterung,
- 292 AD-Wandlung,
- 2921 digitale Filterung,
- 2922 Bestimmung der Signalleistung,
- 2923 Schwellwertbestimmung,
- 293 Segmentierung,
- 2931 Ereigniserkennung,
- 2932 Bestimmung von Periodizität,
- 294 Klassifikation von Rhythmen
- 295 Ende der Wahrnehmung (Messung)

Die Erfindung eliminiert ganz oder teilweise die nachteiligen Wirkungen galvanisch eingekoppelter elektrischer Therapieströme für die Therapie von Herzgewebe, neuronalem Gewebe oder Muskelgewebe.

Dieser selektive Therapieansatz eröffnet neue Möglichkeiten für die multifokale Therapie ohne für jeden Stimulationsort eine gesonderte Sonde implantieren zu müssen. Durch die großflächig mögliche multifokale Therapie können Erregungsmuster angeregt werden, die die natürlichen spatio-temporalen Verhältnisse viel besser als bisher abbilden.

Die Anforderungen an den Energiebedarf von derartigen Implantaten kann erheblich gesenkt werden. Es werden ferner völlig neue Bauformen derartiger Implantate möglich.

Im Kontext der Erfindung werden folgende Begriffe als Synonym für die erfindungsgemäße implantierbare Vorrichtung zur Wahrnehmung von elektromagnetischen Wellen, die von genetisch manipuliertem Gewebe ausgesendet werden, und/oder zur Stimulation von genetisch manipuliertem Gewebe mittels elektromagnetischer Wellen verwendet: Stimulator, Stimulationsgerät, Stimulationsvorrichtung, Vorrichtung/Gerät zur Stimulation, Stimulationssystem (Vorrichtung ist zumindest Teil eines solchen beschriebenen Stimulationssystems).

Im Kontext der Erfindung:
- wird unter "Wellenzug" eine durchgehende elektromagnetische Welle verstanden;
- werden die Begriffe "Elektromagnetische Strahlung", "Elektromagnetische Welle" als Synonym verwendet
- ATP hat die Bedeutung "Anti tachycardia pacing", IPG hat die Bedeutung "Implantable Pulse Generator", ICD hat die Bedeutung "Implantable Cardioverter-Defibrillator"

## Patentansprüche

1. Stimulationsvorrichtung, zumindest umfassend:
eine Energiequelle,
eine Elektronikeinheit,
einen Aktuator, der mit der Elektronikeinheit und/oder der Energiequelle gekoppelt ist,
und wobei die Elektronikeinheit eine Steuereinheit umfasst,
ein Gehäuse, wobei die Energiequelle, die Elektronikeinheit und der Aktuator im Gehäuse angeordnet sind,
eine Fixiereinheit, die mit dem Gehäuse gekoppelt ist, wobei die Fixiereinheit dazu ausgelegt ist, die Stimulationsvorrichtung an einem Herzen oder in einem Herzen zu fixieren,
wobei der Aktuator ausgelegt ist, elektromagnetische Wellen zur Stimulation von genetisch manipuliertem Gewebe auszusenden, und
wobei die Steuereinheit dazu ausgelegt ist, die Stimulation besagten Gewebes mittels der elektromagnetischen Wellen des Aktuators zu steuern,
wobei die Elektronikeinheit eine Aufnahmeeinheit aufweist, die ausgelegt ist zur Messung von Daten, die die Gewebeaktivität und/oder Erfolg einer Stimulation charakterisieren,
wobei die Elektronikeinheit eine Auswerteeinheit aufweist zur Auswertung von Messdaten
hinsichtlich des Erfordernisses einer Stimulation und/oder hinsichtlich des Erfolgs einer Stimulation;
**dadurch gekennzeichnet, dass**
die Elektronikeinheit zusätzlich einen 3D-Beschleunigungssensor umfasst, der zur Therapiekontrolle derart eingesetzt wird, dass nach jeder optischen Stimulation ausgewertet wird, ob eine Beschleunigung der am Myokard fixierten Stimulationsvorrichtung festgestellt wird.

2. Stimulationsvorrichtung nach Anspruch 1, wobei der Aktuator dazu ausgelegt ist, elektromagnetische Wellen im Frequenzspektrum zwischen 10¹³ und 10²⁰ Hz auszusenden.

3. Stimulationsvorrichtung nach mindestens einem der Ansprüche 1 oder 2, wobei das Gehäuse biokompatibles Material umfasst und/oder hermetisch verschlossen ist.

4. Stimulationsvorrichtung nach mindestens einem der vorhergehenden Ansprüche, wobei die Steuereinheit dazu ausgelegt ist, für die Stimulation eine der folgenden Eigenschaften oder eine Kombination der folgenden Eigenschaften zu variieren:
- die Intensität,
- die Frequenz, und/oder
- das Tastverhältnis der elektromagnetischen Wellen.

5. Stimulationsvorrichtung nach Anspruch 4, wobei die Frequenz der Stimulation im Frequenzspektrum zwischen 10¹³ und 10²⁰ Hz liegt.

6. Stimulationsvorrichtung nach mindestens einem der vorigen Ansprüche, wobei die Energiequelle eine Batterie umfasst, die als Primärzelle oder Sekundärzelle gestaltet ist.

7. Stimulationsvorrichtung nach mindestens einem der vorhergehenden Ansprüche, wobei der Aktuator mindestens teilweise mit einem bio-kompatiblen Material beschichtet ist.

8. Stimulationsvorrichtung nach mindestens einem der vorhergehenden Ansprüche, wobei der Aktuator dazu ausgelegt ist, einen lokalen Bereich am oder im Herzen mittels elektromagnetischer Wellen zu stimulieren.

9. Stimulationsvorrichtung nach mindestens einem der vorhergehenden Ansprüche, mindestens aufweisend einen ersten und einen zweiten Aktuator, die dazu ausgelegt sind, unterschiedliche lokale Bereiche am oder im Herzen mittels elektromagnetischer Wellen zu stimulieren.

10. Stimulationsvorrichtung nach mindestens einem der vorhergehenden Ansprüche, wobei die Fixiereinheit und/oder zumindest Teile des Gehäuses mit einer entzündungshemmenden Medikation versehen ist/sind.

11. Stimulationsvorrichtung nach mindestens einem der vorhergehenden Ansprüche, wobei die Vorrichtung eine Einheit zur elektrischen Stimulation des Herzens umfasst.

12. Stimulationsvorrichtung nach mindestens einem der vorigen Ansprüche, wobei die Stimulationsvorrichtung eine Telemetrieeinheit zur Drahtloskommunikation mit mindestens einem externen Gerät und/oder Datenzentrum aufweist.

13. Stimulationsvorrichtung nach mindestens einem der vorigen Ansprüche, wobei die Elektronikeinheit mindestens teilweise durch ein externes Gerät konfigurierbar ist.

14. Stimulationsvorrichtung nach mindestens einem der vorigen Ansprüche, wobei der Aktuator mindestens einen Lichtwellenleiter umfasst.

## Claims

1. Stimulation device, at least comprising:
a source of energy,
an electronic unit,
an actuator coupled to the electronic unit and/or the energy source,
and wherein the electronic unit comprises a control unit,
a housing, wherein the power source, the electronic unit and the actuator are arranged in the housing,
a fixation unit coupled to the housing, the fixation unit configured to fix the stimulation device on or in a heart,
wherein the actuator is configured to emit electromagnetic waves for stimulating genetically manipulated tissue, and
wherein the control unit is configured to control the stimulation of said tissue by means of the electromagnetic waves of the actuator,
wherein the electronic unit comprises a pickup unit configured to measure data characterizing tissue activity and/or success of a stimulation,
wherein the electronic unit comprises an evaluation unit configured to evaluate measurement data with respect to a requirement of a stimulation and/or with respect to a success of a stimulation;
**characterized in that**
the electronic unit further comprises a 3D accelerometer which is used to control of the success of the therapy by evaluating, after every optical stimulation, whether an acceleration of the stimulation device fixed to the myocardium has been detected.

2. Stimulation device according to claim 1, wherein the actuator is configured to emit electromagnetic waves in the frequency spectrum between 10¹³ and 10²⁰ Hz.

3. Stimulation device according to at least one of claims 1 or 2, wherein the housing comprises a biocompatible material and/or is hermetically sealed.

4. Stimulation device according to at least one of the preceding claims, wherein the control unit is configured to vary for the stimulation one of the following properties or a combination of the following properties:
- the intensity,
- the frequency, and/or
- the pulse duty ratio of the electromagnetic waves.

5. Stimulation device of claim 4, wherein the frequency of the stimulation lies in a frequency spectrum between 10¹³ and 10²⁰ Hz.

6. Stimulation device according to at least one of the preceding claims, wherein the energy source comprises a battery configured as a primary cell or a secondary cell.

7. Stimulation device according to at least one of the preceding claims, wherein at least a part of the actuator is coated with a biocompatible material.

8. Stimulation device according to at least one of the preceding claims, wherein the actuator is configured to stimulate a local area on or in the heart by way of electromagnetic waves.

9. Stimulation device according to at least one of the preceding claims, at least comprising a first actuator and a second actuator configured to stimulate different local areas on or in the heart by means of electromagnetic waves.

10. Stimulation device according to at least one of the preceding claims, wherein the fixation unit and/or at least parts of the housing are provided with an antiinflammatory medication.

11. Stimulation device according to at least one of the preceding claims, wherein the device comprises a unit for electrical stimulation of the heart.

12. Stimulation device according to at least one of the preceding claims, wherein the stimulation device comprises a telemetry unit for wireless communication with at least one external device and/or data center.

13. Stimulation device according to at least one of the preceding claims, wherein the electronic unit is at least partly configurable by an external device.

14. Stimulation device according to at least one of the preceding claims, wherein the actuator comprises at least one optical fiber.

## Revendications

1. Dispositif de stimulation, comprenant au moins
une source d'énergie,
une unité électronique,
un actionneur couplé à l'unité électronique et/ou à la source d'énergie,
et dans lequel l'unité électronique comprend une unité de commande,
un boîtier, dans lequel la source d'énergie, l'unité électronique et l'actionneur étant disposés dans le boîtier,
une unité de fixation couplée au boîtier, dans laquelle l'unité de fixation étant conçue pour fixer le dispositif de stimulation à un coeur ou dans un coeur,
dans lequel l'actionneur est adapté pour émettre des ondes électromagnétiques pour stimuler un tissu génétiquement manipulé, et
dans lequel l'unité de commande est adaptée pour commander la stimulation dudit tissu au moyen des ondes électromagnétiques de l'actionneur,
dans lequel l'unité électronique comprend une unité d'enregistrement adaptée pour mesurer des données caractérisant l'activité tissulaire et/ou le succès d'une stimulation,
l'unité électronique présentant une unité d'évaluation pour l'évaluation de données de mesure concernant la nécessité d'une stimulation et/ou concernant le succès d'une stimulation ;
**caractérisé en ce que**
l'unité électronique comprend en outre un capteur d'accélération 3D qui est utilisé pour le contrôle de la thérapie de telle sorte qu'après chaque stimulation optique, on évalue si une accélération du dispositif de stimulation fixé au myocarde est constatée.

2. Dispositif de stimulation selon revendication 1, dans lequel l'actionneur est conçu pour émettre des ondes électromagnétiques dans le spectre de fréquences compris entre 10¹³ et 10²⁰ Hz.

3. Dispositif de stimulation selon au moins l'une des revendications 1 ou 2, dans lequel le boîtier comprend un matériau biocompatible et/ou est fermé hermétiquement.

4. Dispositif de stimulation selon au moins l'une des revendications précédentes, dans lequel l'unité de commande est conçue pour faire varier, pour la stimulation, l'une des caractéristiques suivantes ou une combinaison des caractéristiques suivantes :
- l'intensité,
- la fréquence, et/ou
- le rapport cyclique des ondes électromagnétiques.

5. Dispositif de stimulation selon la revendication 4, dans lequel la fréquence de la stimulation dans le spectre de fréquences est comprise entre 10¹³ et 10²⁰ Hz.

6. Dispositif de stimulation selon au moins l'une des revendications précédentes, dans lequel la source d'énergie comprend une batterie conçue comme un élément primaire ou un élément secondaire.

7. Dispositif de stimulation selon au moins l'une des revendications précédentes, dans lequel l'actionneur est au moins partiellement revêtu d'un matériau biocompatible.

8. Dispositif de stimulation selon au moins l'une des revendications précédentes, dans lequel l'actionneur est adapté pour stimuler une zone locale sur ou dans le coeur au moyen d'ondes électromagnétiques.

9. Dispositif de stimulation selon au moins l'une des revendications précédentes, comprenant au moins un premier et un deuxième actionneurs adaptés pour stimuler différentes zones locales sur ou dans le coeur au moyen d'ondes électromagnétiques.

10. Dispositif de stimulation selon au moins l'une des revendications précédentes, dans lequel l'unité de fixation et/ou au moins des parties du boîtier est/sont pourvue(s) d'un médicament anti-inflammatoire.

11. Dispositif de stimulation selon au moins l'une des revendications précédentes, dans lequel le dispositif comprenant une unité de stimulation électrique du coeur.

12. Dispositif de stimulation selon au moins l'une des revendications précédentes, dans lequel le dispositif de stimulation comprend une unité de télémétrie pour une communication sans fil avec au moins un appareil externe et/ou un centre de données.

13. Dispositif de stimulation selon au moins l'une des revendications précédentes, dans lequel l'unité électronique est au moins partiellement configurable par un appareil externe.

14. Dispositif de stimulation selon au moins l'une des revendications précédentes, dans lequel l'actionneur comprend au moins une fibre optique.
